(19) Europäisches Patentamt
European Patent Office
Office européen des brevets

(11) **EP 0 956 367 B1**

## (12) EUROPÄISCHE PATENTSCHRIFT

(45) Veröffentlichungstag und Bekanntmachung des Hinweises auf die Patenterteilung:
**14.05.2003 Patentblatt 2003/20**

(21) Anmeldenummer: **97945779.3**

(22) Anmeldetag: **21.10.1997**

(51) Int Cl.$^7$: **C12Q 1/68**, G01N 21/64

(86) Internationale Anmeldenummer:
**PCT/DE97/02458**

(87) Internationale Veröffentlichungsnummer:
**WO 98/017824 (30.04.1998 Gazette 1998/17)**

(54) **AUTOMATISIERTE QUANTIFIZIERUNG VON DNA-STRANGBRÜCHEN IN INTAKTEN ZELLEN**

AUTOMATED QUANTIFICATION OF DNA STRAND BREAKS IN INTACT CELLS

QUANTIFICATION AUTOMATISEE DE RUPTURES DE BRINS D'ADN DANS DES CELLULES INTACTES

(84) Benannte Vertragsstaaten:
**AT BE CH DE DK ES FR GB IT LI NL SE**

(30) Priorität: **23.10.1996 DE 19643721**

(43) Veröffentlichungstag der Anmeldung:
**17.11.1999 Patentblatt 1999/46**

(73) Patentinhaber: **Deutsches Krebsforschungszentrum Stiftung des öffentlichen Rechts 69120 Heidelberg (DE)**

(72) Erfinder: **BÜRKLE, Alexander D-69181 Leimen (DE)**

(74) Vertreter: **Schüssler, Andrea, Dr. Kanzlei Huber & Schüssler Truderinger Strasse 246 81825 München (DE)**

(56) Entgegenhaltungen:
EP-A- 0 628 817      DE-A- 3 434 396
US-A- 5 006 460

- BIRNBOIM H C AND JEVCAK J J: "Fluorescence-detected alkaline DNA unwinding" CANCER RESEARCH, Bd. 41, 1981, Seiten 1889-1892, XP002055125 in der Anmeldung erwähnt
- SARAVANAN K ET AL.: "A modified flow injection analysis method to quantify DNA strand-breaks" MEDICAL SCIENCE RESEARCH, Bd. 21, Nr. 14, 1993, Seiten 535-538, XP002055126
- STOUT D L AND BECKER F F: "Fluorometric quantification of single stranded DNA: A method applicable to the technique of alkaline elution" ANALYTICAL BIOCHEMISTRY, Bd. 127, 1982, Seiten 302-307, XP002055127

**Beschreibung**

Die vorliegende Erfindung betrifft ein automatisiertes Verfahren zur Quantifizierung von DNA-Strangbrüchen in intakten Zellen sowie eine Vorrichtung zur Durchführung des Verfahrens.

Der quantitative Nachweis von DNA-Strangbrüchen ist bereits heute für viele Bereiche der biomedizinischen Forschung und Diagnostik von großer Bedeutung. Über den Nachweis von DNA-Strangbrüchen können die Mechanismen von DNA-Schädigung sowie von DNA-Reparatur und ihren Störungen untersucht werden. Weiter ist es damit möglich, ein toxikologisches Screening von Substanzen, wie Chemikalien, Naturstoffen oder Arzneimitteln, durchzuführen. Ferner können mittels Quantifizierung von DNA-Strangbrüchen diverse Zell- und Gewebsproben von Patienten auf DNA-Schädigung bzw. DNA-Reparaturkapazität untersucht werden. Dies ist interessant für das sogenannte Monitoring der Wirksamkeit von Strahlentherapie bzw. zytostatischer Chemotherapie in den abzutötenden malignen Zellen vor und während der Therapie. Es kann auch eine prätherapeutische Abschätzung der Nebenwirkungsrisikos von Strahlentherapie bzw. Chemotherapie durch Untersuchung von normalen (nicht-malignen) Zellen auf DNA-Strangbrüche stattfinden. Die Untersuchung von DNA-Strangbrüchen hat auch eine Bedeutung im Rahmen der Vorsorgemedizin zum Screenen auf Individuen mit hoher Ausprägung von DNA-Schäden bzw. geringer DNA-Reparaturkapazität. Bei diesen Personen muß von einem erhöhten Krebsrisiko ausgegangen werden, so daß für diese ein besonders engmaschiges Vorsorgeprogramm angezeigt ist. In diesem Zusammenhang ist auch an ein sogenanntes Biomonitoring in der Arbeitsmedizin zu denken. Hier würden jeweils standardisierte DNA-Schädigungsbehandlungen an dem zu testenden Probanden-Zellmaterial vorgenommen.

Bisher gibt es einige Methoden zur Untersuchung von DNA-Strangbrüchen, die jedoch alle sehr zeit- und arbeitsaufwendig sind, da sie größtenteils per Hand ausgeführt werden müssen. Ein weiterer Nachteil dieser Methoden ist die unbedingt anzuwendende Sorgfalt bei den diversen Verfahrensschritten, um nicht zu falsch positiven oder falsch negativen Resultaten zu kommen. Als bekannte Methode zur Untersuchung von DNA-Strangbrüchen ist die "alkalische Elution" zu nennen. Hier wird in einem Zellysat eine kontrollierte, alkalische Denaturierung der DNA herbeigeführt. Die Messung der Strangbruchhäufigkeit erfolgt dann über die Bestimmung der Elutionsgeschwindigkeit der einzelsträngigen DNA-Fragmente durch ein geeignetes Polycarbonatfilter. Eine weitere bekannte Methode ist der sogenannte "Kometenassay". Dabei handelt es sich um einen insitu Test, der in verschiedenen Varianten durchgeführt werden kann. Hierbei müssen Zellen in ein Agarosegel eingebettet werden. Nach Lyse "in situ" wird ein elektrisches Feld angelegt, was zu einer mehr oder minder ausgeprägten Wanderung von Chromatin aus dem Kern heraus führt. Die mikroskopisch abzulesende Wanderungsstrecke gilt als Maß für die Anzahl der DNA-Strangbrüche. Bekannt zur Messung von DNA-Strangbrüchen ist außerdem das "Fluorescence-detected Alkaline DNA Unwinding" (Birnboim, H.C. & Jevcak, J.J., Cancer Res. 41, 1889-1892 (1981)), abgekürzt "FADU-Assay". Dabei werden Testzellen lysiert, und die so freigelegte zelluläre DNA, die je nach Vorbehandlung der Zellen eine mehr oder weniger große Anzahl von Einzel- oder Doppelstrangbrüchen aufweist, wird nun unter exakt kontrollierten Bedingungen einer Denaturierung unterzogen, wodurch der DNA-Doppelstrang in Einzelstränge umgewandelt wird. In der Praxis geschieht dies durch extrem vorsichtiges Aufschichten einer alkalischen Lösung, wobei jegliche Vermischung mit der Unterphase (dem Lysat) unbedingt vermieden werden muß. Innerhalb einiger Minuten diffundiert ein Teil der alkalischen Lösung ins Lysat hinein. Die alkalische Denaturierung nimmt dann von DNA-Bruchenden bzw. Chromosomenenden jeweils ihren Ausgang und schreitet linear fort, und zwar bei einem Einzelstrangbruch in beide Richtungen. Nach dem Stoppen dieses alkalischen DNA-Aufschmelzens ("Unwinding") durch Neutralisieren wird der in der Probe verbleibende Rest an nicht-denaturierter DNA über die Intensität der Ethidiumbromid-Fluoreszenz gemessen. Der sich daraus errechnende Anteil denaturierter DNA ist ein direktes Maß für die Anzahl der zum Zeitpunkt der Zell-Lyse vorhandenen DNA-Strangbrüche. Die Fluoreszenzmessung wird normiert (i) auf solche Zellysate (genannt "T-Proben"), bei denen kein denaturierender pH-Wert erreicht wurde, weil der Neutralisierungspuffer vor Zugabe der alkalischen Lösung hinzugegeben worden war, und (ii) auf solche Lysate (genannt "B-Proben"), die vor der alkalischen Denaturierung durch Scheren der DNA (z.B. mittels Ultraschallbehandlung) mit einer sehr großen Anzahl von DNA-Brüchen versehen worden sind. Bei der T-Probe wird der Gehalt an denaturierter DNA gleich 0% gesetzt, während der Gehalt an denaturierter DNA in der B-Probe mit 100% angesetzt wird. Der FADU-Assay ist jedoch sehr arbeitsaufwendig und störanfällig, weil für jede Einzelprobe mehrere hochpräzise Pipettierschritte sowie die exakte Einhaltung von Zeit- und Temperaturbedingungen erforderlich sind. Bei einigen Schritten muß darauf geachtet werden, daß der Inhalt der Probenröhrchen mit mehreren aufeinandergeschichteten Flüssigkeitsphasen nicht durch ein ander gemischt wird. Bei den erforderlichen Vierfach-Parallelbestimmungen, bei den stets mitzuführenden T- und B-Proben und bei einer großen Anzahl von zu bestimmenden Proben summiert sich dies zu einer immensen Pipettierarbeit, wobei jeder Pipettierschritt mit größtmöglicher gleichbleibender Sorgfalt durchzuführen ist. Erschwerend kommt hinzu, daß der FADU-Assay bei Abdunkelung des Labors durchgeführt werden muß, da die Zellysate stark lichtempfindlich sind.

Wie bereits ausgeführt leiden alle diese Methoden unter dem Nachteil, daß sie sehr arbeitsintensiv sind und der Probendurchsatz pro Zeiteinheit nicht sehr hoch ist. So werden für die manuelle Durchführung des FADU-Assays bei 30 Proben ca. 3 Personenarbeitsstunden benötigt. Für die alkalische Elution sind für ca. 36 Proben 16 Stunden (davon 2 Personenarbeitsstunden) zu veranschlagen. Beim Kometenassay werden sogar für die Bearbeitung von nur 20 Proben 8 Personenarbeitsstunden benötigt.

Die Aufgabe der vorliegenden Erfindung besteht deshalb darin, ein Verfahren zur Quantifizierung von DNA-Strangbrüchen bereitzustellen, das einfach durchzuführen ist, einen guten Probendurchsatz pro Zeiteinheit hat und verläßliche Ergebnisse liefert. Die Aufgabe der vorliegenden Erfindung besteht weiter darin, eine Vorrichtung bereitzustellen, mit der das Verfahren durchgeführt werden kann.

Gelöst wird diese Aufgabe durch ein Verfahren gemäß Patentanspruch 1. Weiter wird diese Aufgabe durch eine Vorrichtung gemäß Patentanspruch 5 gelöst. Vorteilhafte Ausgestaltungen ergeben sich aus den Unteransprüchen.

Vom Erfinder wurde der Bedarf erkannt, daß eine Standardisierung der Untersuchung von DNA-Strangbrüchen erfolgen muß. Dies wird durch eine größtmögliche Automatisierung des Verfahrens erreicht.

Das erfindungsgemäße Verfahren zeichnet sich dadurch aus, daß

(a) die Zellen zur Freisetzung von DNA lysiert werden,
(b) das Lysat einer alkalischen Denatunerungsbehandlung unterzogen wird,
(c) eine Neutralisierung stattfindet,
(d) ein Fluoreszenz-Farbstoff zugesetzt wird, und
(e) die Fluoreszenz an einem Fluorometer abgelesen wird, wobei

alle Pipettierschritte automatisiert mittels einer Pipettiereinrichtung in einem lichtdichten Gehäuse ausgeführt werden und vorzugsweise die Fluoreszenzmessungen auch automatisiert ausgeführt werden.

Das erfindungsgemäße Verfahren für die sogenannten "P"-Proben weist vorzugsweise folgende Einzelschritte auf, wobei die Pipettierschritte mittels einer automatischen Pipettiereinrichtung ("Pipettierroboter") ausgeführt werden:

1) Einstellen eines Reaktionsgefäßes, z.B. einer Mikrotiterplatte, enthaltend adhärente Zellen in Zellkulturmedium oder Suspensionszellen in Medium, vorzugsweise in Lösung B, in eine abgedunkelte Pipettierstation
2) bei adhärenten Zellen: Abziehen des Mediums; Inkubationstemperatur 0°C; dann sofort
3) bei adhärenten Zellen: Zugabe von Lösung B (0°C); Inkubationstemperatur 0°C; dann sofort

4) Zugabe von Lösung C (Raumtemperatur); Inkubationstemperatur 0°C; 5-20 Minuten (vorzugsweise 10 Minuten)
5) Zugabe von Lösung D (0°C) (muß verwirbelungsfrei aufgeschichtet werden); Inkubationstemperatur 0°C; dann sofort
6) Zugabe von Lösung E (0°C) (muß verwirbelungsfrei aufgeschichtet werden); Inkubationstemperatur 0°C; 15-45 Minuten (vorzugsweise 30 Minuten)
7) Einstellen der Inkubationstemperatur auf ca. 15°C; 30-90 Minuten (vorzugsweise 60 Minuten)
8) Zugabe von Lösung F (0°C); Mischen; Inkubationstemperatur 0°C; 5-15 Minuten (vorzugsweise 10 Minuten)
9) Scheren der Probe, z.B. durch schnelles Auf- und Abpipettieren oder Ultraschallbehandlung,
10) Zugabe von Lösung G (Raumtemperatur); Inkubationstemperatur ca. 20°C; 5-20 Minuten (vorzugsweise 10 Minuten)
11) Ablesen der Fluoreszenzintensitäten
12) Datenauswertung

Die verwendeten Lösungen sind beispielsweise diejenigen, die in "Birnboim et al, Cancer Res. 41, 1889-1892 (1981)" genannt sind. Im einzelnen sind dies:

Lösung B: 0,25 M meso-Inositol - 10 mM Natriumphosphat - 1 mM MgCl₂ (pH 7,2)
Lösung C: 9 M Harnstoff - 10 mM NaOH - 2,5 mM Cyclohexandiamintetraacetat - 0,1% Natriumdodecylsulfat (= Lysepuffer; Lagerung bei Raumtemperatur)
Lösung D: 0,45 Volumen Lösung C in 0,20 N NaOH (= Alkalische Lösung I)
Lösung E: 0,40 Volumen Lösung C in 0,20 N NaOH (= Alkalische Lösung II)
Lösung F: 1 M Glucose - 14 mM 2-Mercaptoethanol ( = Neutralisierungslösung)
Lösung G: Ethidiumbromid 6,7 µg/ml - 13,3 mM NaOH (Lagerung bei Raumtemperatur)

Die oben angegebenen Zeiten, Temperaturen und Lösungen können natürlich innerhalb bestimmter Grenzen variieren. Diese Variationen liegen im Können des Fachmanns und können mittels Routineversuchen ermittelt werden.

In einer bevorzugten Ausführungsform werden die Lösungen D und E statt in zwei Schritten (Schritte 5) und 6)) kombiniert in einem Schritt appliziert.

Beim Arbeitszyklus für Proben, in denen der Gehalt an denaturierter DNA gleich 0% gesetzt werden kann (vorstehend "T-Proben" genannt), erfolgt Schritt 8) bereits unmittelbar vor Schritt 5). Beim Arbeitszyklus für Proben, in denen der Gehalt an denaturierter DNA gleich 100% gesetzt werden kann (vorstehend "B-Proben" genannt), erfolgt Schritt 9) bereits unmittelbar vor Schritt 7).

Der Prozentsatz D an doppelsträngiger DNA, der sich nach der partiellen Alkalibehandlung noch in der Probe befindet, ist ein Maß für die Anzahl von Strangbrüchen zum Zeitpunkt der Lyse und läßt sich wie folgt berechnen:

$$D (\%) = (P - B) ./. (T - B) \times 100$$

Anstelle von Ethidiumbromid, das bekanntlich mutagen ist, kann in Lösung G prinzipiell auch ein anderer, nicht-toxischer Fluoreszenzfarbstoff, der in doppelsträngige DNA interkaliert (z.B. Sybr-Green™), verwendet werden.

Mit dem Begriff "adhärente Zellen" sind solche Zellen gemeint, die in einem Reaktionsgefäß, z.B. auf einer Mikrotiterplatte, als Monolayer in Zellkulturmedium angezüchtet werden können. Als Zellkulturmedium eignen sich die dem Fachmann bekannten käuflichen Medien, die je nach anzuwachsendem Zelltyp ausgewählt werden. Einige Stunden bis ca. einen Tag später können die auf dem Reaktionsgefäß angewachsenen Zellen direkt in die Vorrichtung gestellt werden. Beispiele für adhärente Zellen sind Fibroblasten, HeLa-Zellen und die meisten Tumorzellen.

Es ist allerdings auch möglich, "Suspensionszellen" einzusetzen. Suspensionszellen sind Zellen, die man nicht auf Zellkulturplatten als Monolayer züchten kann. Die Suspensionszellen müssen zuerst aus dem entsprechenden Medium abzentrifugiert werden und dann in einem Puffer, z.B. in Lösung B, resuspendiert werden, bevor sie in ein Reaktionsgefäß, z.B. eine Mikrotiterplatte, eingefüllt werden können und nach obigem Schema untersucht werden können. Beispiele für Suspensionszellen sind Blutzellen, wie Leukozyten, oder lymphoide Zellinien.

In einer bevorzugten Ausführungsform kann das erfindungsgemäße Verfahren auch auf die Quantifizierung von bestimmten Schäden an DNA-Basen ausgeweitet werden. Dazu wird das Zellysat, das mit einem Lysepuffer behandelt worden ist, der weniger Harnstoff als Lösung C enthält, vor der alkalischen Denaturierung einer Inkubation mit einer gereinigten DNA-Reparatur-Endonuklease unterzogen, welche hochspezifisch bestimmte DNA-Schädigungen (z.B. 8-Oxa-Guanin bzw. Pyrimidin-Dimere) erkennt und lokal in die betroffenen DNA-Stränge einschneidet, so daß für jede geschädigte Base nun ein DNA-Strangbruch entsteht. Diese in vitro generierten, zusätzlichen Brüche lassen sich dann unmittelbar anschließend mit dem erfindungsgemäßen Meßverfahren bestimmen. Ein Beispiel für eine solche Endonuklease ist das sogenannte "FAPY-Enzym". Damit läßt sich der Anwendungsbereich der erfindungsgemäßen Verfahrens erheblich ausweiten, nämlich auf die hochspezifische Quantifizierung von unreparierten primären Schäden an DNA-Basen.

Bei der erfindungsgemäßen Vorrichtung handelt es sich im Prinzip um einen Pipettierroboter, der über ein lichtdichtes Gehäuse verfügt. Das Reaktionsgefäß, vorzugsweise die Mikrotiterplatte, wird darin auf eine temperierbare Basisplatte gestellt, die die Proben auf Temperaturen von 0°C bis 30°C bringen kann. Die Vorrichtung verfügt über ein System von Pipettierdüsen zur Entnahme bzw. zum Hinzufügen von Flüssigkeiten aus dem bzw. in das Reaktionsgefäß, vorzugsweise die Mikrotiterplatte. Ein weiterer Bestandteil der Vorrichtung ist eine Fluorometer-Einrichtung mit einer Anregungswellenlänge von 490-550 nm, vorzugsweise 520 nm, und einer Emissionswellenlänge von 570-610 nm, vorzugsweise 590 nm. Das Fluorometer ist so ausgestattet, daß die Fluoreszenzintensität in jeder Vertiefung der bevorzugten Mikrotiterplatte parallel oder sequentiell "in situ" abgelesen werden kann. Unter den Begriff "Fluorometer" fällt auch eine einfache Einrichtung, die unter Anwendung einer Lichtquelle und zwei verschiedenden Filtern die Beobachtung des Fluoreszenzphänomens zuläßt. Vorzugsweise verfügt die erfindungsgemäße Vorrichtung weiter über einen Mikroprozessor und einen Printer bzw. Plotter.

Vorteile des erfindungsgemäßen Verfahrens sind, daß unter Einsatz des erfindungsgemäßen Geräts eine Mikrotiterplatte mit 96 Meßpunkten in weniger als 2 Gerätearbeitsstunden zu bearbeiten ist. Die Personenarbeitszeit beträgt dagegen nur noch einige Minuten verglichen mit den oben genannten Zeiten für die manuelle Untersuchung von DNA-Strangbrüchen. Außerdem kann mit dem erfindungsgemäßen Verfahren die Zellyse direkt mit adhärenten Einschicht-Zellkulturen erfolgen. Letzteres ist deswegen besonders interessant, weil dadurch die vorherige Ablösung von Zellen von ihrer Unterlage (z.B. mit Trypsin) nicht notwendig ist. Damit entfallen entsprechende Zentrifugierschritte, die (i) zeit- und kostenträchtig sind und (ii) artifiziell bereits zur Induktion von DNA-Brüchen führen können bzw. das DNA-Reparaturverhalten der Zelle ungünstig beeinflussen. Somit bietet das erfindungsgemäße Verfahren den zusätzlichen Vorteil, DNA-Schädigung und Reparatur unverfälscht messen zu können.

Die Erfindung wird durch das nachfolgende Beispiel weiter erläutert:

## BEISPIEL

HeLa-Zellen werden mit DMEM-Medium (Dulbelcco's modified essential medium) plus 10% fötales Kälberserum in die Vertiefungen einer für die Zellkultur geeigneten Mikrotiterplatte gegeben und für 12 Stunden inkubiert, wobei die Zellen vollständig anwachsen. Danach werden die intakten Zellen bei 0°C (auf Eis) einer radioaktiven Strahlung ($^{60}$Co $\gamma$-Strahlung) ausgesetzt. Danach wird das erfindungsgemäße Verfahren wie folgt durchgeführt:

1) Einstellen der Mikrotiterplatte in die abgedunkelte Pipettierstation

2) Abziehen des Mediums; Temperatur der Basisplatte 0°C; dann sofort

3) Zugabe von 40 µl Lösung B (0°C); Temperatur der Basisplatte 0°C; dann sofort

4) Zugabe von 40 µl Lösung C (Raumtemperatur); Temperatur der Basisplatte 0°C; Inkubation 10 Minuten

5) Zugabe von 20 µl Lösung D (0°C) (muß verwirbelungsfrei aufgeschichtet werden); Temperatur der Basisplatte 0°C; dann sofort

6) Zugabe von Lösung 20 µl Lösung E (0°C) (muß verwirbelungsfrei aufgeschichtet werden); Temperatur der Basisplatte 0°C; 15-45 Minuten (vorzugsweise 30 Minuten)

7) Aufheizen der Basisplatte auf ca. 15°C; 60 Minuten inkubieren

8) Zugabe von 80 µl Lösung F (0°C); Mischen; Temperatur der Basisplatte 0°C; 10 Minuten inkubieren

9) Scheren der Probe durch schnelles Auf- und Abpipettieren

10) Entnahme von 100 µl und Transfer auf eine neue Mikrotiterplatte

11) Zugabe von 150 µl Lösung G (Raumtemperatur); Temperatur der Basisplatte 20°C; 10 Minuten inkubieren

12) Ablesen der Fluoreszenzintensitäten anhand des eingebauten Fluorometers (Anregungswellenlänge 520 nm; Emissionswellenlänge 590 nm)

13) Datenauswertung

Die Zusammensetzungen der Lösungen B bis G sind wie vorstehend angegeben.

Beim Arbeitszyklus für Proben, in denen der Gehalt an denaturierter DNA gleich 0% gesetzt werden soll (vorstehend "T-Proben" genannt), erfolgt Schritt 8) bereits unmittelbar vor Schritt 5). Beim Arbeitszyklus für Proben, in denen der Gehalt an denaturierter DNA gleich 100% gesetzt werden kann (vorstehend "B-Proben" genannt), erfolgt Schritt 9) bereits unmittelbar vor Schritt 7).

Um die Verlässlichkeit des Verfahrens zu überprüfen, wurden alle Werte vierfach bestimmt und die Abweichung voneinander berechnet. Diese Datenauswertung ergab, daß die Bestimmungen verläßliche Werte ergaben, wobei die Abweichungen der Vierfachbestimmungen um 2 % lagen.

**Patentansprüche**

1. Verfahren zur Quantifizierung von DNA-Strangbrüchen in intakten Zellen, wobei

(a) die Zellen zur Freisetzung von DNA lysiert werden,

(b) das Lysat einer alkalischen Denaturierungsbehandlung unterzogen wird,

(c) eine Neutralisierung stattfindet,

(d) ein Fluoreszenz-Farbstoff zugesetzt wird, und

(e) die Fluoreszenz an einem Fluorometer abgelesen wird,

**dadurch gekennzeichnet, daß** alle Pipettierschritte automatisiert mittels einer Pipettiereinrichtung in einem lichtdichten Gehäuse ausgeführt werden.

2. Verfahren nach Anspruch 1, **dadurch gekennzeichnet, daß** das Verfahren in Mikrotiterplatten stattfindet.

3. Verfahren nach Anspruch 1 oder 2, **dadurch gekennzeichnet, daß** der Fluoreszenz-Farbstoff in doppelsträngige DNA interkaliert, insbesondere Ethidiumbromid ist.

4. Verfahren nach einem der Ansprüche 1 bis 3, **dadurch gekennzeichnet, daß** die Fluoreszenz bei einer Anregungswellenlänge von 520 nm und einer Emissionswellenlänge von 590 nm gemessen wird.

5. Vorrichtung zur Durchführung des Verfahrens nach einem der Ansprüche 1 bis 4, **gekennzeichnet durch** ein lichtdichtes Gehäuse, eine temperierbare Basisplatte, ein System von Pipettierdüsen und ein Fluorometer zum Messen der Fluoreszenzintensität von DNA.

6. Vorrichtung nach Anspruch 5, **dadurch gekennzeichnet, daß** sie weiter einen Mikroprozessor und einen Printer und/oder Plotter aufweist.

7. Vorrichtung nach Anspruch 5 oder 6, **dadurch gekennzeichnet, daß** die temperierbare Basisplatte auf Temperaturen von 0 bis 30°C regelbar ist.

8. Vorrichtung nach einem der Ansprüche 5 bis 7, **dadurch gekennzeichnet, daß** das Fluorometer bei einer Anregungswellenlänge von 520 nm und einer Emissionswellenlänge von 590 nm mißt.

**Claims**

1. A method of quantifying DNA strand breaks in intact cells, wherein

(a) the cells are lysed to release DNA,

(b) the lysate is subjected to an alkaline denaturation treatment,

(c) neutralization is effected,

(d) a fluorescent dye is added, and

(e) fluorescence is read off a fluorometer,

**characterized in that** all pipetting steps are carried out in automated fashion by means of a pipetting device in a lightproof housing.

**2.** The method according to claim 1, **characterized in that** the method takes place in microtiter plates.

**3.** The method according to claim 1 or 2, **characterized in that** the fluorescent dye is intercalated in doublestranded DNA, particularly it is ethidium bromide.

**4.** The method according to any one of claims 1 to 3, **characterized in that** the fluorescence is measured at an excitation wavelength of 520 nm and an emission wavelength of 590 nm.

**5.** A device for carrying out said method according to any one of claims 1 to 4, **characterized by** a lightproof housing, a temperature controllable base plate, a system of pipetting nozzles and a fluorometer for measuring the fluorescence intensity of DNA.

**6.** The device according to claim 5, **characterized in that** it further comprises a microprocessor and a printer and/or plotter.

**7.** The device according to claim 5 or 6, **characterized in that** the temperature controllable base plate is adjustable to temperatures from 0 to 30°C.

**8.** The device according to any one of claims 5 to 7, **characterized in that** the fluorometer measures at an excitation wavelength of 520 nm and an emission wavelength of 590 nm.

## Revendications

**1.** Méthode de quantification automatique de cassures de brins d'ADN dans des cellules intactes, dans laquelle

(a) les cellules sont lysées pour libérer l'ADN,
(b) le lysat est soumis à un traitement alcalin de dénaturation,
(c) une neutralisation a lieu,
(d) un colorant fluorescent est ajouté, et
(e) la fluorescence est lue sur un fluorimètre,

**caractérisée en ce que** toutes étapes de pipetage rendues automatiques au moyen d'un dispositif de pipetage sont conduites dans un boîtier opaque.

**2.** Méthode suivant la revendication 1, **caractérisée en ce qu'**elle est mise en oeuvre dans des plaques de microtitrage.

**3.** Méthode suivant la revendication 1 ou 2, **caractérisée en ce que** le colorant fluorescent intercalé dans l'ADN double-brin est en particulier le bromure d'éthidium.

**4.** Méthode suivant l'une des revendications 1 à 3, **caractérisée en ce que** la fluorescence est mesurée à une longueur d'onde d'excitation de 520 nm et à une longueur d'onde d'émission de 590 nm.

**5.** Dispositif pour la mise en oeuvre de la méthode suivant l'une des revendications 1 à 4, **caractérisé en ce qu'**il comprend un boîtier opaque, une plaque de base pouvant être amenée à température constante, un système de buses de pipetage et un fluorimètre destiné à la mesure de l'intensité de fluorescence de l'ADN.

**6.** Dispositif suivant la revendication 5, **caractérisé en ce qu'**il comprend en outre un microprocesseur et une imprimante et/ou un traceur de courbes.

**7.** Dispositif suivant la revendication 5 ou 6, **caractérisé en ce que** la plaque de base pouvant être amenée à température constante est réglable à des températures de 0 à 30°C.

**8.** Dispositif suivant l'une des revendications 5 à 7, **caractérisé en ce que** le fluorimètre mesure à une longueur d'onde d'excitation de 520 nm et à une longueur d'onde d'émission de 590 nm.